# EUROPEAN PATENT APPLICATION

(11) **EP 0 730 065 A2**
(43) Date of publication of application: **04.09.1996**
(21) Application number: 96103161.4
(22) Date of filing: 01.03.1996
(51) Int. Cl.: E03D 9/04

(54) **Device for freshening the air adjacent to toilets**

(30) Priority: 02.03.1995 IL 11286795
(71) Applicant: RAZ PITUACH MOTZARIM (1995) LTD., Raanana 43558 (IL)
(72) Inventor: Edry, Rafi, Eilat 88000 (IL)
(74) Representative: Weisert, Annekäte, Dipl.-Ing. Dr.-Ing.

(57) **Abstract**

A device for freshening ambient air of a toilet having a toilet flushing system including a water tank, by mounting a housing containing an air-freshener body adjacent to the water tank of the toilet flushing system, and circulating ambient air, which is drawn into the water tank during flushing and/or expelled from the water tank during refilling, through the housing in contact with the air-freshener body and out through the housing into the ambient air.

## Description

The present invention relates to a device for freshening the air adjacent to toilets.

Many techniques have been proposed for freshening the air adjacent to toilets. Some techniques involve passing the air from the toilet bowl through filters. Other proposed techniques involve introducing an air-freshener substance into the air continuously, periodically, or each time the toilet is used. Among the latter techniques there have been proposed air-freshener devices which are actuated by opening or closing the toilet door, by sitting on the toilet seat, and the like. None of these techniques appears to have found widespread use probably because of the cost or complexity of the device, or of the manner of installing the device.

According to the invention, there is provided a device for freshening the ambient air of a toilet having a toilet flushing system including a water tank, comprising: a housing mountable adjacent to the water tank of the toilet flushing system; an air-freshener body within the housing; and a fluid circuit for circulating through the housing, in contact with the air-freshener body therein and out through the housing, ambient air drawn into the water tank during the flushing of the water therefrom and/or air expelled from the water tank during the refilling thereof with water.

As will be described more particularly below, the device for freshening the air in accordance with the foregoing features of the present invention may be implemented in a relatively simple structure which can be manufactured at low cost and which can be mounted to existing water tanks in a very simple and convenient manner.
Fig. 1 illustrates one form of air-freshener device constructed in accordance with the present invention;
Fig. 2 is an enlarged, fragmentary, sectional view illustrating the device of Fig. 1;
Fig. 3 is a side elevational view illustrating a modification in the construction of the air-freshener device;
Fig. 4 is a sectional view illustrating a second form of air-freshener device constructed in accordance with the present invention; and
Fig. 5 is a sectional view along line V--V of Fig. 4.

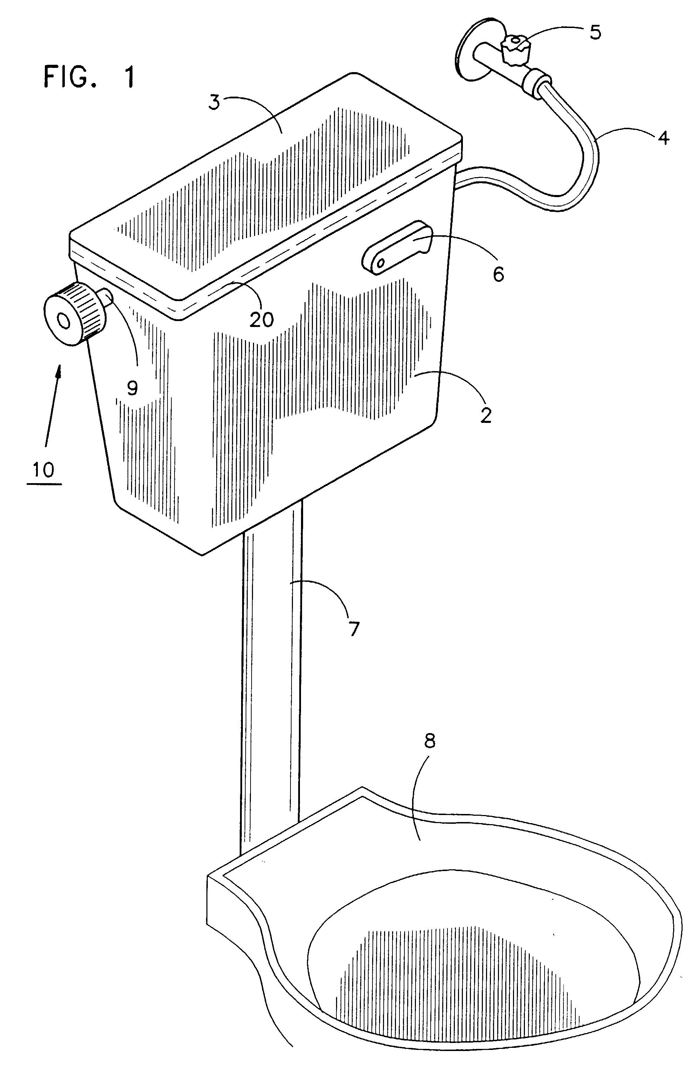

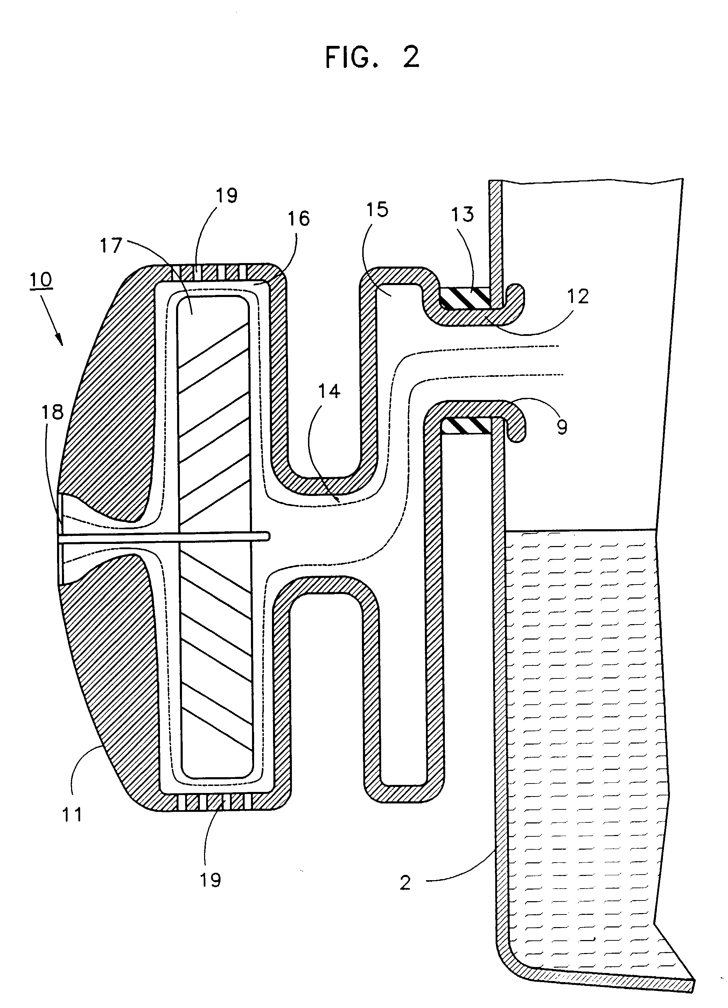

Figs. 1 and 2 illustrate a water tank 2 as used in a conventional toilet flushing system. The water tank 2 is closed by a lid 3 and is supplied with water from the supply mains 4 under the control of a manual valve 5. The tank further includes a lever 6 which is manually pivotted to actuate a flush valve (not shown) within the tank to flush the water via a tube 7 into the toilet bowl 8.

Such water tanks 2 are commonly provided with two openings on its opposite sides for connection to the water supply pipe 4. This enables the water supply pipe to be connected either from one side or the other side of the tank according to the particular situation at the installation site. Thus, one opening is used for connection to the water supply pipe 4, whereas the other opening is normally plugged.

The air-freshener device illustrated in Figs. 1 and 2 utilizes the second opening in the water supply tank, designated 9 in Figs. 1 and 2, normally not used for the water supply pipe 4 and therefore normally plugged, for attaching the air-freshener device, generally designated 10.

The air-freshener device 10, as more particularly illustrated in Fig. 2, comprises a housing 11 integrally formed with a cylindrical mounting fitting 12 for mounting the housing within opening 9 of the water tank 2. Fitting 12 further includes a sealing ring 13 for sealing the connection of the housing to the water supply tank.

Housing 11 is of generally cylindrical configuration but is reduced in diameter at a mid-portion defining a restrictor passageway 14 connecting a first housing chamber 15 to a second housing chamber 16. Chamber 16 receives an air-freshener body 17 also of substantially cylindrical configuration. Housing 11 is formed with a large opening 18 extending axially of the air-freshener body 17, and a plurality of small openings 19 extending through the circumferential wall of the housing around the air-freshener body.

It will be seen that the mounting fitting 12 of the air-freshener device 10 establishes communication between the interior of the upper end of the water tank 2 and the interior of housing 11. Thus, when the water tank 2 is flushed, and the water from the tank is emptied into the toilet bowl, air is drawn via openings 18 and 19 through housing 11 of the air-freshener device 10 and into the upper end of the water tank. The air drawn into the water tank flows around the air-freshener body 17 within housing chamber 16 and thereby becomes loaded with the air-freshener substance of that body. Thus, at the end of the flushing operation when water tank 2 is substantially empty of water, the water tank is filled with ambient air loaded with the water freshener substance of body 17.

As the empty tank 2 starts to refill from the water supply pipe 4, the air within the tank, loaded with the air-freshener susbtance, is expelled via fitting 12, chamber 15, restrictor passageway 14, and chamber 16, again around body 17, and out through openings 18 and 19 in housing 11, thereby freshening the air in the immediate area.

Restrictor passageway 14 increases the velocity of the air as it passes around the air-freshener body 17. The volume of chamber 16 is only slightly larger than the volume of the air-freshener body 17, so that a large surface area of body 17 is exposed to the air passing through that chamber.

It will thus be seen that, in the device illustrated in Figs. 1 and 2, the air-freshener body 17 is exposed to ambient air both during the flushing phase, when the water tank 2 is filled with air by the emptying of the water from the tank, and also during the refilling phase when the air within the water tank is expelled by the refilling of the water tank with water. Normally, this exposure of the air-freshener body to the air is sufficient despite some air leakage between the lid 3 and the water supply tank 2. However, if it is desired to increase the exposure of the air-freshener body to the air, a sealing ring, shown schematically at 20 in Fig. 1, may be provided between the upper end of the water supply tank 2 and the lid 3.

The air-freshener device 10 may be constructed as a disposable unit to be replaced when the air-freshener body 17 has been substantially exhausted. Alternatively, it may be constructed as a refillable unit in which the air-freshener body 17 alone is to be replaced by a new body. This may be done by forming the respective end wall of the housing 11 as a removable cap enabling it to be removed, to provide access into chamber 16 for removing and exhausted air-freshener body and replacing it with a fresh one.

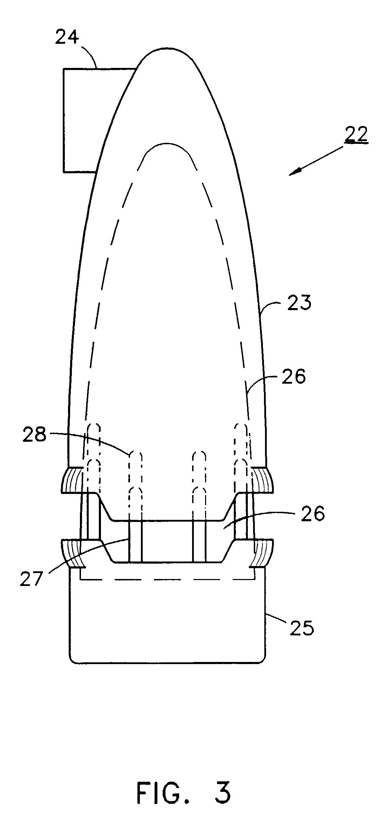

Fig. 3 illustrates another construction that may be used for the air-freshener device, therein designated 22. Here, the device includes a housing 23 integrally formed at its upper end with cylindrical fitting 24 for mounting the housing within opening 9 of the water tank, and a removable base 25 at its lower end carrying the air-freshener body 26.

Body 25 is integrally formed with a plurality of ribs 27 received within recesses 28 formed in the inner surface of housing section 23. Ribs 27 and recesses 28 are dimensioned such that the base 25 can be spaced from the lower end of housing section 23 to provide the air passageways for the ambient air to enter the housing during the flushing of the water, or to be expelled from the housing during the refilling of the tank. Ribs 27 and recess 28 also permit base 25 to be moved to a closed position against the lower end of housing 23, in order to prevent dissipation of the air-freshener body 26. The arrangement illustrated in Fig. 2a thus permits the air freshener body 26 to be viewed through the openings between ribs 27 and to be replaced by a refill unit including base 25 and a fresh body 26. It also permits these air circulating openings to be closed in order to preserve the air-freshener body.

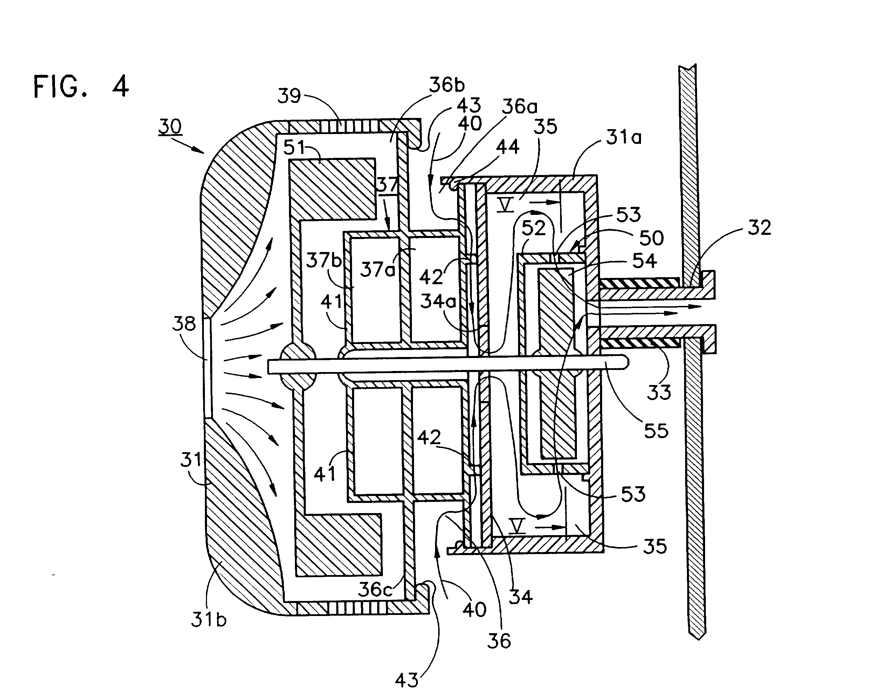

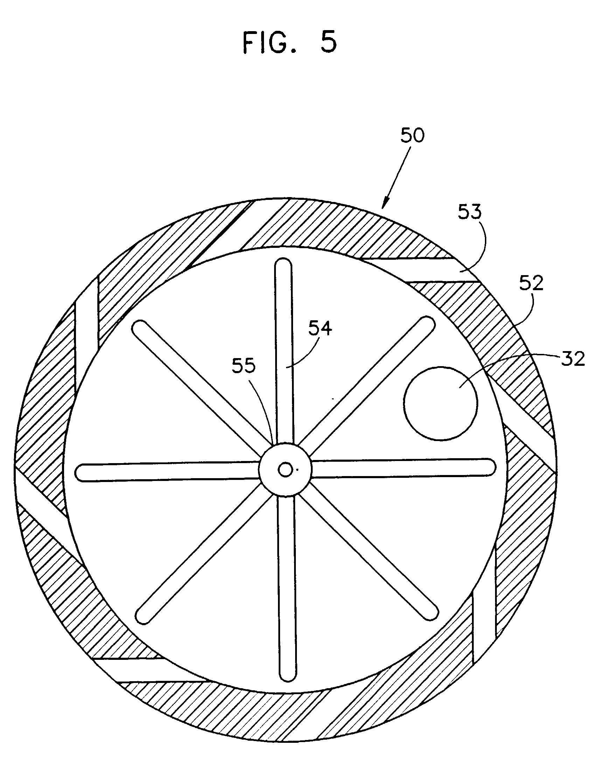

Figs. 4 and 5 illustrate a further form of air-freshener device, generally designated 30, constructed in accordance with the invention which may also be attached to the water tank 2 of a toilet flushing system for automatically freshening the air upon the actuation of the flushing system. The air-freshener device 30 illustrated in Figs. 3 and 4 also includes a housing 31 integrally formed at one end with a cylindrical mounting fitting 32 for mounting the device within opening 9 (Fig. 1) of the water tank, in order to establish communication between the interior of the housing 31 and the upper end of the water tank. Fitting 32 includes a sealing ring 33 for sealing this connection.

The interior of housing 31 is divided by a wall 34 into a first chamber 35 adjacent to fitting 32, and a second chamber 36 containing the air-freshener body 37. Chamber 36, however, is divided into two sub-chambers or sections 36a, 36b, by a partition wall 36c. The air-freshener body 37 is also divided by partition wall 36c into a first section 37a within chamber section 36a, and a second section 37b within chamber section 36b.

Housing 31 is also formed in two sections, namely a small-diameter section 31a adjacent to the mounting fitting 32 and defining chamber 35, and a large-diameter section 31b received over the outer circumference of partition wall 36c. Thus, housing section 31b and the partition wall 36c define the chamber section 36b receiving the air-freshener body section 37b. The end wall of housing section 31b is formed with a large opening 38 extending axially with respect to the air-freshener body, and a plurality of smaller openings 39 extending around section 37b of the air-freshener body.

The small-diameter section 31a of housing 31 terminates short of partition wall 36c so as to define an annular opening 40 between the two housing sections and communicating with chamber section 36a of the housing receiving the air-freshener body section 37a.

The two sections 37a, 37b of the air-freshener body 37 are enclosed within a screen 41 permeable by air to expose the air-freshener susbstance within body 37 to the air flowing through chamber sections 36a and 36b. The air-freshener body 37, including its screen enclosure 41, is carried by partition wall 36c, and is provided with a plurality of axially-extending, circumferentially-spaced spacer elements 42 for spacing the screen enclosure 41 from partition wall 34.

Housing section 31b is formed with an annular rib 43 on its inner surface engageable with wall 36c for mounting that housing section to the wall. Screen enclosure 41 of the air-freshener body 37 includes an annular ring engageable with an annular rib 44 formed on the inner surface of housing section 31a for securing wall 36c, and the air-freshener body 37 carried thereby, to housing section 31a.

Chamber 35 adjacent to fitting 32 mounting the device to the water tank includes an impeller, generally designated 50, for rotating a fan 51 located within chamber section 36b. Fan 51 force-circulates the air from opening 38 around the air-freshener body section 37b and out through openings 39.

As shown in Fig. 5, impeller 50 includes a cylindrical housing 52 formed with a plurality of tangential openings 53 to produce a cyclonic flow of the air through the interior of housing 52 to impinge a plurality of impeller vanes 54. These vanes are fixed to a shaft 55 extending axially of the housing and coupled to the fan blades 51 within chamber section 36b at the opposite end of the housing for force-circulating the air through the air-freshener body section 37b and out through openings 39.

The device illustrated in Figs. 4 and 5 operates as follows:

During the flushing phase, when the water is flushed from the water tank (2, Fig. 1), the emptying of the water from the tank draws air into the upper end of the tank via annular opening 40 in housing 31, restrictor space 34a, tangential openings 53 of the impeller 50, and mounting fitting 32 communicating with the interior of the upper end of the water tank. This flow of air comes into direct contact with section 37a of air-freshener body 37, and thereby loads the air with the air-freshener substance of that body, as described above with respect to Figs. 1 and 2. This flow of air, however, also impinges vanes 54 to rotate the impeller shaft 55, and thereby the fan blades 51. Therefore, the fan blades force-circulate air from housing opening 38 past the air-freshener body section 37b and out through openings 39 to the atmosphere, thereby freshening the air during this flushing phase.

During the refilling phase, the rising level of the water forces the air within the upper end of the tank, previously loaded with the air-freshener substance during the flushing phase, to flow via fitting 32 through the turbine 50, its openings 53, restrictor passage 34a, around the air-freshener body section 37a, and out through the annular opening 40 from the housing, thereby discharging refreshed air also during the refilling phase.

In the construction illustrated in Figs. 4 and 5, the fan 51 is not rotated during this refilling phase. However, it will be appreciated that by forming the passageway through fitting 32 also as a tangential opening to produce a cyclonic flow in the reverse flow direction, fan 51 may also be driven during the refilling phase to force-circulate the air past air-freshener body section 37b and out through openings 39.

## Claims

1. A device for freshening the ambient air of a toilet having a toilet flushing system including a water tank, comprising:
a housing mountable adjacent to the water tank of the toilet flushing system;
an air-freshener body within said housing;
and a fluid circuit for circulating through said housing, in contact with the air-freshener body therein and out through the housing, ambient air drawn into the water tank during the flushing of the water therefrom and/or air expelled from the water tank during the refilling thereof with water.

2. The device according to Claim 1, wherein said fluid circuit circulates through said housing, in contact with the air-freshener body therein, both the ambient air drawn into the water tank during the flushing of the water therefrom, and the air expelled from the water tank during the refilling thereof with water.

3. The device according to Claim 2, wherein said fluid circuit includes a mounting fitting for mounting the housing to the water tank, said mounting fitting including a passageway therethrough establishing fluid communication between the interior of the housing and the interior of the upper end of the water tank.

4. The device according to Claim 3, wherein said fluid circuit further includes a plurality of openings in the housing wall, which openings serve both for inletting ambient air into the housing when the ambient air is drawn into the water tank during the flushing of water therefrom, and for outletting air from the housing when the water is expelled from the water tank during its refilling with water.

5. The device according to Claim 4, wherein said air-freshener body is located in a chamber between said openings and said mounting fitting, said chamber being of slightly larger volume than said air-freshener body to provide an increased surface of exposure of the air-freshener body to the air circulated therethrough.

6. The device according to Claim 1, wherein said housing includes a fan driven by the air drawn into the water tank during the flushing of the water therefrom, for force-circulating air into contact with the air-freshener body.

7. The device according to Claim 6, wherein said housing includes a first section containing a first section of the air-freshener body which is contacted by the air drawn into the water tank during the flushing of the water therefrom, and also by the air expelled from the water tank during the refilling thereof; and a second section containing a second section of the air-freshener body and also containing said fan which is driven by the air drawn into the water tank during the flushing of the water therefrom to force-circulate air with respect to said other section of the air-freshener body.

8. The device according to Claim 7, wherein said fan is coupled to a turbine in said first section of the housing carried by the air drawn into the water tank during the flushing of the water therefrom.

9. The device according to Claim 8, wherein said turbine is located in a chamber adjacent to said mounting fitting and formed with a plurality of tangential openings for producing a cyclonic flow of the air into said chamber.

10. The device according to Claim 9, wherein said turbine includes a plurality of radially-extending vanes secured to a shaft coupled to said fan, said tangential openings being formed to rotate the vanes and shaft by the air when drawn into the water tank.
